# EUROPÄISCHE PATENTSCHRIFT

(11) **EP 0 926 155 B1**
(45) Veröffentlichungstag und Bekanntmachung des Hinweises auf die Patenterteilung: **15.02.2006**
(21) Anmeldenummer: 98122545.1
(22) Anmeldetag: 01.12.1998
(51) Int. Cl.: C07K 14/045, A61K 38/16, G01N 33/68, C07K 16/08, C07K 14/05

(54) **Polypeptide mit Aminosäuresequenzen aus dem N-terminalen Bereich von gp116 von Cytomegalovirus und deren Verwendung bei der Diagnostik, Prophylaxe und Therapie**
Polypeptides from the N-terminal domain of gp116 of cytomegalovirus and their diagnostic, prophylactic and therapeutic use
Polypeptides du domaine N-terminal de la protéine gp116 du cytomegalovirus et leur utilisation diagnostique, prophylactique et therapeutique

(30) Priorität: 17.12.1997 DE 19756214
(43) Veröffentlichungstag der Anmeldung: 30.06.1999
(73) Patentinhaber: Biotest AG, D-63303 Dreieich (DE)
(72) Erfinder: Hinderer, Walter, Dr., 63110 Rodgau (DE); Lang, Dieter, Dr., 63128 Dietzenbach (DE); Rothe, Markus, Dr., 63073 Offenbach/M. (DE); Vornhagen, Rolf, Dr., 63225 Langen (DE)
(74) Vertreter: Keller, Günter

(56) Entgegenhaltungen:
- WO-A-93/21952
- AYATA MINORU ET AL: "Different antibody response to a neutralizing epitope of human cytomegalovirus glycoprotein B among seropositive individuals." JOURNAL OF MEDICAL VIROLOGY, Bd. 43, Nr. 4, 1994, Seiten 386-392, XP001070205 ISSN: 0146-6615
- KROPFF BARBARA ET AL: "An ELISA using recombinant proteins for the detection of neutralizing antibodies against human cytomegalovirus." JOURNAL OF MEDICAL VIROLOGY, Bd. 39, Nr. 3, 1993, Seiten 187-195, XP002196603 ISSN: 0146-6615
- BERENCSI K ET AL: "The N-terminal 303 amino acids of the human cytomegalovirus envelope glycoprotein B (UL55) and the exon 4 region of the major immediate early protein 1 (UL123) induce a cytotoxic T-cell response" VACCINE, BUTTERWORTH SCIENTIFIC. GUILDFORD, GB, Bd. 14, Nr. 5, 1. April 1996 (1996-04-01), Seiten 369-374, XP004057290 ISSN: 0264-410X
- MEYER H ET AL: "THE GP116 OF THE GP58-116 COMPLEX OF HUMAN CYTOMEGALOVIRUS REPRESENTS THE AMINO-TERMINAL PART OF THE PRECURSOR MOLECULE AND CONTAINS A NEUTRALIZING EPITOPE" JOURNAL OF GENERAL VIROLOGY, Bd. 71, Nr. 10, 1990, Seiten 2443-2450, XP001070655 ISSN: 0022-1317
- SUHRBIER A: "Multi-epitope DNA vaccines." IMMUNOLOGY AND CELL BIOLOGY, Bd. 75, Nr. 4, 1997, Seiten 402-408, XP000867239 ISSN: 0818-9641

## Beschreibung

Die vorliegende Erfindung betrifft bestimmte Polypeptide aus dem N-terminalen Bereich des in der Virushülle des humanen Cytomegalovirus (HCMV) vorkommenden Glykoproteins mit dem Molekulargewicht von 116 kD (gp116) sowie deren Verwendung in Diagnostik, Prophylaxe und Therapie.

Das HCMV stellt den Prototyp der β-Herpesviren dar, die durch ihre Wirts-Spezifität und durch ihren langen Replikationszyklus charakterisiert sind. Hierbei handelt es sich um ein sehr verbreitetes Virus, da etwa 50 % der Bevölkerung in Europa nachweisbare Serumantikörper gegen HCMV besitzen. Üblicherweise erfolgt die Infektion mit HCMV im Kindes- bzw. Jugendalter und verläuft bei immunkompetenten Individuen in der Regel inapparent. In seltenen Fällen kann sich das Krankheitsbild einer infektiösen Mononukleose (Pfeiffer'sches Drüsenfieber) manifestieren.

Infektionen mit HCMV stellen bei bestimmten Patientengruppen ein ernsthaftes Gesundheitsproblem dar. Eine HCMV-Infektion kann bei Schwangeren, Neugeborenen, Transplantatempfängern und AIDS-Patienten erhebliche Probleme verursachen, ja sogar lebensbedrohend werden.

Eine HCMV-Infektion ist bei diesen Patientengruppen von erheblicher klinischer Bedeutung. Bei Patienten mit erworbener Immunschwäche (AIDS) stellt HCMV die häufigste opportunistische Infektion dar, die mit einer schwerwiegenden Symptomatik einhergeht. Patienten, die eine solide Organtransplantation oder eine allogene Knochenmarkstransplantation erhalten, werden chemotherapeutisch immunsupprimiert. Dadurch kann es zu schweren, oft tödlichen Infektionsverläufen mit HCMV kommen, insbesondere dann, wenn diese Patienten nicht über Antikörper gegen HCMV verfügen.

Besonderes Augenmerk gilt auch der HCMV-Infektion während der Schwangerschaft. Etwa 10 % der kongenital infizierten Neugeborenen leiden unter Leber-Milz-Vergrößerung oder Gehörschäden sowie Lernschwächen, die in der frühen Kindesentwicklung auftreten. Dabei liegt die Wahrscheinlichkeit für Mütter mit primärer HCMV-Infektion, das Virus auf den Fötus zu übertragen, erheblich höher (etwa 40 %) als für Mütter mit sekundärer Infektion (etwa 1 %). Neben dem verringerten Übertragungsrisiko sind auch die Auswirkungen einer HCMV-Übertragung auf das Neugeborene nach Zweitkontakt der Mutter mit dem Virus erheblich vermindert. Offensichtlich kann die zu einem früheren Zeitpunkt erworbene Immunität der Mutter das Neugeborene vor einer symptomatischen Infektion schützen. Die Bildung von virusneutralisierenden Antikörpern (nAk) durch das mütterliche Immunsystem ist hierbei von ganz besonderer Bedeutung. Man kann davon ausgehen, daß nAk den Ausbruch einer HCMV-Infektion nach rekurrierender HCMV-Infektion während der Schwangerschaft verhindern können.

Ayata et al. [Journal of Medical Virology (1994), Seiten 386-392] beschreiben Antikörper, die gegenüber der Peptidsequenz des Glykoproteins B spezifisch sind.

Kropff et al. [Journal of Medical Virology (1993), Seiten 187-195] beschreiben die Verwendung von rekombinanten Proteinen für den Nachweis von neutralisierenden Antikörpern gegen das humane Cytomegalovirus.

Ein Aspekt der vorliegenden Erfindung betrifft die Bereitstellung von Polypeptiden mit HCMV-spezifischen Sequenzen, die für die Diagnostik, Prophylaxe und Therapie von HCMV-Infektionen vorteilhafterweise verwendet werden können.

Bei einer initialen Infektion mit HCMV werden Antikörper sowohl gegen die Struktur- als auch gegen die Nichtstrukturproteine gebildet. Diese Antikörper erscheinen relativ früh nach der Infektion (gut nachweisbar etwa ab Tag 14), besitzen aber keinen neutralisierenden Charakter. Neutralisierende Antikörper, die vor allem gegen die Glykoproteine der Virushülle gerichtet sind, werden relativ spät nach dem Erstkontakt mit dem Virus (etwa ab dem 100. Tag) gebildet. Diese neutralisierenden Antikörper sind essentieller Bestandteil einer lebenslangen Immunität gegen HCMV. Bei einer Reaktivierung oder bei einer sekundären Superinfektion mit HCMV werden diese neutralisierenden Antikörper sofort gebildet, während eine verzögerte Bildung von neutralisierenden Antikörpern auf eine primäre HCMV-Infektion hinweist. Der spezifische Nachweis von nAk wird daher im Rahmen der vorliegenden Erfindung eingesetzt, um bei den interessierenden Patientengruppen zwischen primärer und sekundärer HCMV-Infektion unterscheiden zu können. Diese Unterscheidung zwischen primärer und sekundärer HCMV-Infektion ist besonders wichtig während einer Schwangerschaft und nach Transplantation, da hiervon weiterführende diagnostische oder therapeutische Maßnahmen abhängen.

Eine prophylaktische Möglichkeit, die Symptome der HCMV-Infektion bei Transplantat-Empfängern zu mildern, ist die Gabe von Immunglobulinpräparaten mit CMV-spezifischen Antikörpern. Obwohl durch Studien noch nicht eindeutig belegt, geht man davon aus, daß die neutralisierenden Antikörper die entscheidende Komponente für die Wirksamkeit darstellen. Daher ist es wesentlich, daß die Immunglobulinpräparate spezifisch auf ihren Gehalt an Antikörpern gegen HCMV und insbesondere an neutralisierenden HCMV-Antikörpern getestet werden können. Derartige Präparate können bei den gefährdeten Patientengruppen zu effizienten Therapieerfolgen führen.

Die vorliegende Erfindung betrifft bestimmte Bereiche am N-terminalen Ende des Glykoproteins gp116 von HCMV. Der Glykoproteinkomplex gp58/116 ist homolog zu dem Glykoprotein B (gB) bei Herpes simplex Viren. Der Komplex besteht aus zwei Polypeptiden, einem hoch glykosilierten Protein von 90 kD bis 130 kD und einem weniger stark modifizierten Protein mit etwa 58 kD. Die über Disulfidbrücken verbundenen Komponenten stammen von einem glykosilierten Vorläufermolekül mit einer Größe von 150 kD bis 160 kD ab, das proteolytisch gespalten wird, und zwar etwa bei der Position 460 des primären Translationsproduktes. Die Spaltung erfolgt durch eine zelluläre Endoprotease. Gp58 stellt hierbei den C-terminalen Teil des Vorläuferproteins dar; wahrscheinlich hat es die Funktion eines Transmembranproteins.

Das Vorläuferprotein von gp58/116 weist etwa 906-907 Aminosäuren (abhängig vom HCMV-Stamm) auf und wird durch das Gen mit der Bezeichnung UL55 kodiert.

Durch Immunoblotanalysen konnte gezeigt werden, daß die Mehrzahl von humanen, HCMV-seropositiven Seren mit gp58-116 (im folgenden kurz gB) reagieren und daß 40-70 % der neutralisierenden Kapazität dieser Seren gegen gB gerichtet ist. Durch Epitopanalyse mit neutralisierenden anti-gB-spezifischen monoklonalen Antikörpern konnten im wesentlichen zwei antigene Domänen identifiziert werden. Die als AD1 bezeichnete antigene Domäne wurde auf einer kontinuierlichen Sequenz von mehr als 70 Aminosäuren zwischen den Aminosäurepositionen 552 und 635 von gp58 lokalisiert (Wagner et al., Journal of Virology, September 1992, S. 5290-5297).

Als weitere antigene Domäne wurde AD2 identifiziert, die sich etwa zwischen den Aminosäurepositionen 27 und 100 des Glykoproteins gp116 befindet (Kropff et al., Journal of Medical Virology 39 (1993), S. 187-195).

Die antigene Domäne AD2 umfaßt wenigstens zwei unterschiedliche Epitope. Das eine Epitop befindet sich in dem Bereich zwischen Aminosäureposition 24 und 67 und zeichnet sich durch eine hohe Sequenzvariabilität innerhalb verschiedener HCMV-Stämme aus und kann daher als stammspezifisches Epitop angesehen werden.

Die Nukleotidsequenzen von zwei bevorzugt eingesetzten HCMV-Stämmen mit der Bezeichnung "AD169" und "Towne" wurden bereits publiziert (Cranage et al., EMBO J., 5 (1986), S. 3057-3063 bzw. Spaete et al., Virology, 167 (1988), S. 207-225). Vergleicht man die Sequenzen dieser beiden Stämme miteinander, so stellt man fest, daß der eine Epitop-Bereich zwischen Aminosäure 24 und 67 stark unterschiedlich ist, wohingegen der andere Epitopbereich zwischen Aminosäure 67 und 78 sehr konserviert ist. Dieses Epitop induziert die Bildung von neutralisierenden Antikörpern.

Gegenstand der vorliegenden Erfindung sind daher Polypeptide gemäß Anspruch 1 mit humanem Cytomegalievirus (HCMV) spezifischen Sequenzen, die dadurch gekennzeichnet sind, daß jedes Polypeptid wenigstens zwei Polypeptidfragmente umfaßt, wobei jedes Fragment die Aminosäuresequenz einer homologen Region aus dem N-terminalen Bereich des Glykoproteins gp116 aufweist.

Die erfindungsgemäßen Polypeptide weisen Aminosäuresequenzen auf, die einem Bereich am N-Terminus des Glykoproteins gp116 entsprechen. Hervorzuheben ist, daß die erfindungsgemäßen Polypeptide zwei homologe Regionen, d.h. einander entsprechende Regionen aus diesem N-terminalen Bereich des Glykoproteins gp116 aufweisen, wobei die eine Region von einem bestimmten HCMV-Stamm herstammt, der die Bezeichnung AD169 hat, wohingegen die andere Region von einem anderen HCMV-Stamm herstammt, der die Bezeichnung "Towne" hat.

Bei der erfindungsgemäß eingesetzten Region aus dem N-terminalen Bereich handelt es sich um den sogenannten AD2-Bereich.

Die erfindungsgemäßen Polypeptide weisen also zwei Polypeptidfragmente auf, wobei jedes Fragment den AD2-Bereich eines HCMV-Stammes aufweist. Außer den beiden genannten HCMV-Stämmen "AD169" und "Towne" können selbstverständlich noch andere HCMV-Stämme eingesetzt werden, zusätzlich zu den genannten Stämmen "AD169" und "Towne".

Das eine bevorzugte Polypeptidfragment, das die Aminosäuresequenz des AD2-Bereichs des HCMV-Stammes AD169 beinhaltet, umfaßt die folgende Aminosäuresequenz mit der Sequenz-ID-Nr. 1:

STSHATSSTHNGSHTSRTTSAQTRSVYSQHVTSSEAVSHRANETIYNTTLKYGDVVGV.
(Seq.-ID-Nr. 1)

Die erfindungsgemäß bevorzugt eingesetzte homologe Region aus dem Stamm Towne umfaßt die als Sequenz-ID-Nr. 2 bezeichnete Aminosäuresequenz:

STRGTSATHSHHSSHTTSAAHSRSGSVSQRVTSSQTVSHGVNETIYN.
(Seq.-ID-Nr. 2)

Die erfindungsgemäßen Polypeptide weisen wenigstens zwei Polypeptidfragmente auf, wobei jedes dieser Polypeptidfragmente jeweils eine homologe Region aus dem N-terminalen Bereich von gp116 beinhaltet. Die einzelnen Polypeptidfragmente können in Form einer Mischung, bevorzugt im Verhältnis 1:5, vorliegen, allerdings ist auch eine Mischung von 1:10 bis 10:1 möglich. Alternativ hierzu können die Polypeptidfragmente auch kovalent miteinander verbunden sein.

Die erfindungsgemäßen Polypeptide können mit Hilfe an sich wohlbekannter Methoden chemisch synthetisiert werden (beispielsweise Festphasensynthese). Alternativ hierzu können die erfindungsgemäßen Polypeptide auch mit Hilfe der Gentechnologie in Wirtsorganismen, bevorzugt Hefen oder Bakterien, insbesondere E.coli, rekombinant hergestellt werden. Je nach Einsatzzweck können die rekombinant hergestellten Polypeptide in Form von heterologen Fusionsproteinen oder in Form von autologen Fusionsproteinen vorliegen. Bei den heterologen Fusionsproteinen stammt der heterologe Teil vom Vektor ab. Hierbei kann es sich beispielsweise um die Glutation-S-Transferase oder einen Teil davon handeln. Bei den autologen Fusionsproteinen sind die entsprechenden codierenden DNA-Sequenzen fusioniert und die Polypeptidfragmente werden zusammen als ein Polypeptid exprimiert.

Schließlich ist es auch möglich, daß die einzelnen Polypeptidfragmente mit Hilfe einer chemischen Koppelung miteinander verbunden werden. Hierbei kann es sich um eine direkte Bindung handeln oder um die Verbindung mittels eines Brückenmoleküls, das an den Enden wenigstens zwei reaktive Gruppen aufweist, die mit den einzelnen Polypeptidfragmenten Bindungen ausbilden können.

Die erfindungsgemäßen Polypeptide werden bevorzugt in der Diagnostik eingesetzt. Hierzu finden die Polypeptide Verwendung in an sich bekannten Testkits, die zum Nachweis von Antikörpern gegen HCMV geeignet sind. Derartige Testkits umfassen an sich übliche Testbestandteile, wobei die einzelnen Bestandteile von der jeweiligen Testdurchführung abhängt. Es kann sich beispielsweise um Kits zur Durchführung von Western Blots, von RIA- oder von ELISA-Tests handeln. Diesen Tests gemeinsam ist, daß mit Hilfe des erfindungsgemäßen Polypeptids spezielle Antikörper, insbesondere schützende Antikörper gegen HCMV durch Reaktion des Polypeptids mit den Antikörpern nachgewiesen werden können.

In einer besonders bevorzugten Ausführungsform werden die erfindungsgemäßen Polypeptide in einem Testkit zur Durchführung des ELISA-Tests eingesetzt. Ein derartiges Testkit umfaßt die an sich üblichen Bestandteile, wie vorbereitete Testmikrotiterplatten und die erforderlichen Reagenzien zur Durchführung des ELISA-Tests.

In einer bevorzugten Ausführungsform betrifft die Erfindung einen gB ELISA, bei dem die antigenen Domänen AD2 der HCMV Stämme AD169 und Towne eingesetzt werden. Der ELISA basiert auf dem indirekten Testprinzip zum Nachweis von IgG Antikörpern und verwendet ein anti-human-IgG-Peroxidasekonjugat. Die feste Phase wurde mit einem gB Peptid (AS 27-84) des HCMV Stamms AD169 und mit einem gB Peptid (27-73) des HCMV Stammes Towne beschichtet.

Im Gegensatz zu veröffentlichten Daten (Kropff et al. a.a.O.), konnte mit der antigenen Domäne AD1 von gB keine zufriedenstellende Reaktivität erreicht werden, so daß nur der N-terminale Teil von gB im Test verwendet wurde. Überraschenderweise konnte gezeigt werden, daß das gB aus AD169 alleine nicht ausreichte, um eine zufriedenstellende Sensitivität zu vermitteln. Nur durch die Ergänzung mit einem weiteren gB-spezifischen Peptid eines zweiten HCMV-Stamms konnte die Sensitivität des Tests in den verschiedenen Serumkollektiven gesteigert werden. Dieser Umstand weist darauf hin, daß Individuen aus der normalen Bevölkerung mit Towne-ähnlichen HCMV-Stämmen infiziert sind. Die Seren einiger Individuen reagieren nicht kreuz mit der AD2-Region von gB AD169, können jedoch mit einer Towne-spezifischen Testkomponente nachgewiesen werden. Die Kombination von wenigstens zwei stammspezifischen Peptiden aus dem N-terminalen Teil von gB ermöglicht deutlich verbesserte Testergebnisse. Der Test ermöglicht die Detektion von neutralisierenden Antikörpern und kann in der Schwangeren- und Transplantationsdiagnostik zur Diskriminierung zwischen akuter und rekurrierender HCMV-Infektion eingesetzt werden. Die Höhe der OD-Werte im Test korrelierte mit der Höhe des Gehalts an neutralisierenden Antikörpern (n-AK-Titer). Neben der diagnostischen Anwendung bei den Patientengruppen, die durch eine HCMV-Infektion besonders bedroht sind, können die Polypeptide bzw. die diese enthaltenden Testkits zum Untersuchen von Plasmaspenden eingesetzt werden, um die geeigneten Proben für die Herstellung von Hyperimmunglobulinpräparaten auszuwählen. Bei den Hyperimmunglobulinpräparaten handelt es sich um solche Präparate, die besonders reich an Anti-HCMV-Antikörpern sind, wobei ein hoher Anteil von neutralisierenden Antikörpern als besonders wichtig für die Therapie von HCMV-Infektionen angesehen wird.

Der Test eignet sich demnach insbesondere zur Identifizierung hochtitriger Seren oder Plasmen aus Spenderproben zur Herstellung von anti HCMV spezifischen Immunglobulinpräparaten und zum Vergleich derselben.

Ein anderer Aspekt der vorliegenden Erfindung betrifft die Verwendung der erfindungsgemäßen Polypeptide für die Herstellung von Impfstoffen. Die Herstellung von Impfstoffen ist in der Fachwelt wohlbekannt. Der Einsatz von Polypeptiden anstelle von atenuierten Virusstämmen beinhaltet einige Vorteile, insbesondere dann, wenn durch die eingesetzten Polypeptide neutralisierende Antikörper bei dem geimpften Patienten hervorgerufen werden können. Die erfindungsgemäßen Polypeptide können entweder allein oder in Kombination mit anderen Polypeptiden, die ebenfalls neutralisierende Antikörper hervorrufen, für die Herstellung eines Impfstoffes eingesetzt werden.

Ein weiterer Aspekt der vorliegenden Erfindung betrifft neutralisierende, gegen HCMV gerichtete Antikörper. In einer bevorzugten Ausführungsform handelt es sich bei derartigen Antikörpern um humane oder humanisierte monoklonale Antikörper, die spezifisch an ein erfindungsgemäßes Polypeptid binden. Die Herstellung von humanisierten monoklonalen Antikörpern ist dem Fachmann grundsätzlich bekannt. Hierbei werden zunächst in einem Tier, beispielsweise einer Maus oder Ratte, monoklonale Antikörper gegen die Polypeptide hergestellt. Hierbei handelt es sich um ein Standardverfahren, das auf die Arbeiten von Köhler und Millstein zurückgeht.

Wenn ein entsprechender monoklonaler Antikörper aufgefunden wurde, der gut an das Polypeptid bindet und, der auch eine neutralisierende Wirkung zeigt, wird aus diesem monoklonalen Antikörper der für die bindende Region des Antikörpers kodierende Bereich isoliert und mit Hilfe der rekombinanten Technik in einen menschlichen Antikörper eingebaut. Ein derartiger humanisierter monoklonaler Antikörper ist daher ein auf einen menschlichen Antikörper zurückgehender Antikörper, bei dem lediglich die bindenden Regionen von einem in einer anderen Spezies erzeugten Antikörper herstammen.

Alternativ können auch Antikörper produzierende, humane Zellklone mit Hilfe der erfindungsgemäßen Polypeptide auf neutralisierende Antikörper untersucht werden. Mit Hilfe von dem Fachmann bekannten Immortalisierungsverfahren, wie z.B. der EBV-Transformation, gelangt man zu Zellklonen, die permanent die gewünschten monoklonalen Antikörper synthetisieren.

Besonders bevorzugt wird auch ein Antikörpergemisch, das wenigstens zwei verschiedene humane oder humanisierte monoklonale Antikörper umfaßt, wobei bevorzugt der eine Antikörper gerichtet ist gegen ein Epitop der AD2-Region des HCMV-Stamms AD169 und der andere Antikörper gerichtet ist gegen ein Epitop der AD2-Region vom HCMV-Stamm Towne.

Die vorliegende Erfindung wird durch die nachfolgenden Beispiele näher erläutert. Um die Übersichtlichkeit der Abkürzungen zu gewährleisten, werden diese in der nachfolgenden Tabelle 1 schematisch zusammengefaßt:

### Übersicht der untersuchten gB-Antigene

**Tabelle 1: Übersicht der erfindungsgemäß eingesetzten gB-Antigene**

| **Antigen** | **CMV- Protein** | **Stamm** | **Aminosäuren** | **Herstellung** |
|---|---|---|---|---|
| AD1-GST | gp58 | AD169 | 545-679 | rekombinant, heterologe Fusion |
| AD1 | gp58 | AD169 | 549-630 | synthetisches Peptid |
| AD2-GST | gp116 | AD169 | 27-84 | rekombinant, heterologe Fusion |
| AD2-GST | gp116 | Towne | 27-88 | rekombinant, heterologe Fusion |
| AD2 | gp116 | AD169 | 27-84 | synthetisches Peptid |
| AD2 | gp116 | Towne | 27-73 | synthetisches Peptid |
| AD1+AD2 | gp58/116 | AD169 | 27-84 545-679 | rekombinant, autologe Fusion |
| AD2+AD2-GST | gp116 | AD169 +Towne | 27-84 27-88 | rekombinant,autologe und heterologe Fusion |
| AD2+AD2 | gp116 | AD169 +Towne | 27-84 27-88 | rekombinant, autologe Fusion |

### Beispiel 1

### Klonierung der antigenen Domänen AD1 und AD2 von HCMV Stamm AD169 bzw. Towne

### a) Autologe Fusionen

Ausgehend von genomischer HCMV DNA wurden mittels sequenzspezifischer DNA-Primer (siehe Tabelle 2) die entsprechenden antigenen Domänen von HCMV gB durch Polymerase Ketten Reaktion (PCR) amplifiziert. Die Durchführung der PCR sowie die Weiterverarbeitung der Amplifikate durch Restriktionsverdau Analyse, Ligation, Transformation und Plasmid-DNA Screening erfolgte mit den bekannten Standard Methoden (Maniatis et al. 1989). Zunächst wurde mit den Primern pccAD21 und pccAD22 (HCMV Stamm AD169) eine gerichtete PCR durchgeführt. Durch die überhängenden Nukleotide der Primer wurden in das PCR-Amplifikat am 3'-Ende ein BamHl- und am 5'-Ende eine BglII- und eine EcoR1 Schnittstelle eingefügt. Durch Restriktionsverdau des PCR-Produkts mit BamH1/EcoR1 lies sich das Primäramplifikat in den Standardvektor PUC8 einbringen, der ebenfalls mit BamH1/EcoR1 vorverdaut und präpariert worden war. Der Vektor wurde mit pUC8AD2 bezeichnet. Nach Behandlung des Vektors pUC8AD2 mit BglII und EcoR1 konnten dann weitere Antigenkomponenten C-terminal von AD2 eingefügt werden, da die am 5'-Ende eingefügte BglII Schnittstelle eine Klonierung innerhalb des Leserasters erlaubte. Unter Verwendung der Primerpaare pccAD1/pccG55/2 und AD21/AD22 (HCMV Stamm Towne) wurden die DNA-Fragmente der antigenen Domänen AD1 (AD169) und AD2 (Towne) amplifiziert und mit den Restriktionsenzymen BamH1 und EcoR1 verdaut. Diese modifizierten PCR-Producte wurden dann mit dem Vektor pUC8AD2, der mit BgIII/EcoR1 verdaut und präpariert worden war, in einer Ligationsreaktion eingesetzt. Aufgrund der Kompatibilität der Schnittstellen von BglII und BamH1 war eine autologe Fusion von AD2 (AD169) mit AD1(AD169) bzw von AD2 (AD169) mit AD2 (Towne) innerhalb des Leserasters möglich. Zur Expression der autologen Fusionsproteine AD2/AD1 und AD2/AD2 wurden die DNA-Frangmente mittels BamH1/EcoR1 Verdau aus dem Vektor pUC8 in die Standard Expressionsvektoren pET5c oder pGEX3* umgesetzt.

### b) GST-Fusionsproteine der Einzelantigene

Die Klonierung in pGEX3* ermöglichte die Expression eines Gluthathion-S-Transferase Fusionsproteins, welches in ELISA und Western Blot Analysen eingesetzt werden konnte. Dazu wurden ausgehend von genomischer HCMV DNA mittels sequenzspezifischer DNA-Primer (siehe Tabelle 2) die entsprechenden DNA-Fragmente der antigenen Domänen AD2 (AD169), AD1 (AD169) und AD2 (Towne) durch Polymerase Ketten Reaktion (PCR) amplifiziert. Die Durchführung der PCR sowie die Weiterverarbeitung der Amplifikate mit Restriktionsverdau Analyse, Ligation, Transformation und Plasmid-DNA Screening erfolgte mit den bekannten Standard Methoden (Maniatis et al. 1989). Durch die überhängenden Nukleotide der primer wurden in das PCR-Amplifikat am 3'-Ende ein BamH1 und am 5'-Ende eine BglII und eine EcoR1 Schnittstelle eingefügt. Durch Restriktionsverdau der jeweiligen PCR-Produkte mit BamH1/EcoR1 liesen sich die Primäramplifikate in den Standardvektor pGEX3* einbringen, der ebenfalls mit BamH1/EcoR1 vorverdaut und präpariert worden war.

### Tabelle der PCR primer für die Klonierung

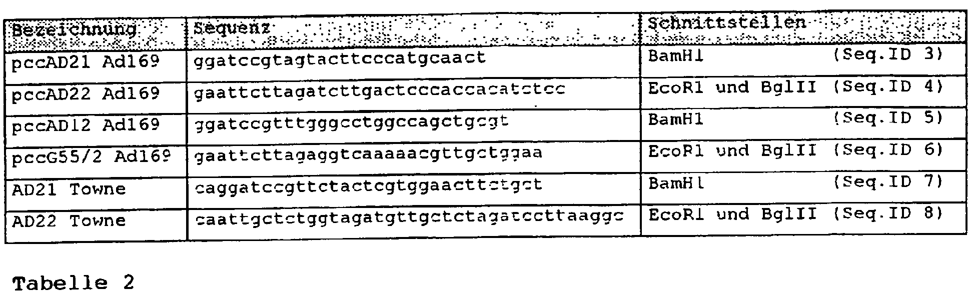

### Beispiel 2

### Anzucht, Expression und Reinigung des heterologen Fusionsproteins AD2-GST

Mit einer isolierten Kolonie auf einer Agarplatte des Klons pGEX-3/pCCAD2/JM109 (LB/AMP Medium) wurden 150 ml LB/AMP-Medium angeimpft. Die Kultivierung erfolgte in einem 1 1 EMK, bei 37°, im Rundschüttler bei 160 Upm für 16 h.

Die Anzucht der 3 1 Kultur erfolgte in 6 Parallelansätzen zu je 0.5 1 in 2 1 EMK mit Schikanen. Das Medium (LB/,AMP) war auf 37° vortemperiert. Die Kolben wurden inokuliert mit je 20 ml der Vorkultur (1:26) und inkubiert bei 37°C und 160 Upm im Rundschüttler. Das Wachstum wurde kontinuierlich verfolgt durch Messung der Absorption A bei 600 nm. Bei einer A (600 nm) von 0.7 wurde induziert durch Zugabe von IPTG (Endkonz. 1 mM).

Die Ernte erfolgte 4 h nach Induktion durch Zentrifugation (6x1 1-Becher, 4000 g, 0-4°C, 30 min). Die gut abgetropften Bakterienpellets wurden in 200 ml eiskaltem PBS resuspendiert und erneut zentrifugiert (2x250ml-Becher, 5000 g, 0-4°, 10 min). Die gut abgetropften Pellets wurden eingefroren und gelagert bei -20 bis -30°C.

Nach Auftauen der Bakterienpellets wurden diese in 80 ml Basis-Puffer (Tris-HCl/20mM/pH7.5) resuspendiert und anschließend homogenisiert (Teflon/Glas-Potter-Homogenisator). Unter Rühren bei Raumtemperatur wurden folgende Additive zugegeben: NP-40 (0.1 %), PMSF (0.1 mM), Pefabloc (0.1 mM), EDTA (50 mM) und Lysozym (100 mg). Das Gesamtvolumen betrug 100 ml. Der Ansatz wurde bei Raumtemperatur stark gerührt und nach 60 min auf Eis gestellt. Alle weiteren Schritte erfolgten auf Eis bzw. gekühlt. Nach der Inkubation wurde Glycerin (10 %) und 2-Mercaptoethanol (14 mM) zugegeben und vermischt. Das Volumen wurde mit Basis-Puffer auf 140 ml eingestellt. Der Lyseansatz wurde anschließend sonifiziert (20 kHz, pulsierend, 5 min, Titanrüssel 3/4"), gefolgt von maschinellem Pottern (Teflon/Glas-Potter-Homogenisator, 1000 UpM, 10 Stöße). Nicht-solubilisiertes Material wurde durch Zentrifugation abgetrennt (4 x 50 ml-Becher, 40000 g, 30 min, 0-4°C) und verworfen.

Das lösliche Protein im Überstand wurde über GSH-Sepharose-4B (Pharmacia) chromatographiert. Dabei wurde eine Säule der Dimension 2.6 x 10 cm (30 ml) verwendet. A (280 nm) wurde kontinuierlich registriert. Die Flußrate betrug 2 ml/min. Der Säulenpuffer war Tris-HCl/20 mM/pH7.5 mit 1.4 mM Mercaptoethanol. Nach erfolgtem Probenauftrag wurde solange mit Säulenpuffer nachgespült, bis der Schreiber die Basislinie wieder erreicht hatte. Anschließend wurden 50 ml Waschpuffer, Tris-HCl/100 mM/pH9 mit 10 % Glycerin, 0.5 % NP-40 und 1.4 mM Mercaptoethanol über die Säule gegeben gefolgt von Säulenpuffer. Nachdem der Schreiber wieder die Basislinie erreicht hatte, wurde das gebundene GST-Protein mit 5 mM Glutathion (GSHred.) in Säulenpuffer eluiert. Das GSH wurde unmittelbar vor der Verwendung gelöst. Das GSH-Eluat (vom Schreiber registrierter Absorptionspeak) wurde gesammelt und aliquotiert, eingefroren und gelagert bei -60 bis -80°C.

Die Anzucht, Expression und Reinigung von Glutathion S-transferase (GST Kontrollprotein) erfolgte in gleicher Weise wie für das autologe Fusionsprotein AD2-GST oben beschrieben.

### Beispiel 3

### Anzucht, Expression und Reinigung des intakten (27k) autologen Fusionsproteins AD1+2

Ausgehend von einer Plattenkolonie (LB/CA,AMP) des Klons pET5c/pCCAD1+2/ BL21(DE3)pLysS wurde eine 15 ml Kultur (LB/CA, AMP) bei 37°C im Rundschüttler bis zu einer A (600 nm) = 1.8-2.0 angezogen. Die Kultur wurde dann vermischt mit Glycerin (87 %), bis zu einer Endkonzentration von 15 % (v/v). Die Kultur wurde in 0.1 ml- Portionen eingefroren und gelagert bei -60 bis -80°C. Ein gefrorenes Aliquot der Vorkultur wurde rasch aufgetaut und in 150 ml LB/CA, AMP-Medium einpipettiert. Die Kultivierung erfolgte in einem 1 1 EMK, bei 28°C, im Rundschüttler bei 100 Upm, für 16 h.

Die Anzucht der 6 1 Kultur erfolgte in 12 Parallelansätzen zu je 0.5 1 in 2 1 EMK mit Schikanen. Das Medium (LB/CA, AMP) war auf 37°C vortemperiert. Die Kolben wurden inokuliert mit je 10 ml der Vorkultur (1:51) und inkubiert bei 37°C und 160 Upm im Rundschüttler. Das Wachstum wurde kontinuierlich verfolgt, durch Messung von A (600 nm). Bei einer A (600 nm) von 0.6 wurde induziert, durch Zugabe von IPTG (Endkonz. 1 mM).

Die Ernte erfolgte 3 h nach Induktion durch Zentrifugation (6 x 1 1-Becher, 4000 g, 0-4°C, 30 min). Die gut abgetropften Bakterienpellets wurden in 200 ml eiskaltem PBS resuspendiert und erneut zentrifugiert (2 x 250 ml-Becher, 5000 g, 0-4°C, 10 min). Die gut abgetropften Pellets wurden eingefroren und gelagert bei -20 bis -30°C.

Die eingefrorenen Bakterienpellets wurden aufgetaut und resuspendiert in 160 ml Basis-Puffer (Tris-HCl/20 mM/pH7.5) und anschließend mit einem (Teflon/Glas-Potter-Homogenisator) homogenisiert. Unter Rühren bei Raumtemperatur wurden folgende Additive zugegeben: NP-40 (0.05 %), PMSF (0.2 mM), Pefabloc (0.2 mM), EDTA (50 mM) und Lysozym (50 mg). Das Gesamtvolumen betrug 200 ml. Der Ansatz wurde nach Lysozymzugabe sofort auf Eis gestellt. Alle weiteren Schritte erfolgten auf Eis bzw. gekühlt. Nach der Inkubation wurde Glycerin (10 %) und 2-Mercaptoethanol (14 mM) zugegeben und vermischt. Das Volumen wurde mit Basis-Puffer auf 280 ml eingestellt. Der Lyseansatz wurde anschließend sonifiziert (20 kHz, pulsierend, 5 min, Titanrüssel 3/4"), gefolgt von maschinellem Pottern (Teflon/Glas-Potter-Homogenisator, 1000 UpM, 10 Stöße). Solubilisiertes Material wurde durch Zentrifugation entfernt (2 x 250 ml-Becher, 15000 g, 10 min, 0-4°C). Die Überstände wurden verworfen.

Die Pellets wurden gut abgetropft und sofort resuspendiert in 100 ml Tris-HCl/100 mM/pH9 mit 10 % Glycerin, 0.5 % NP-40 und 14 mM Mercaptoethanol und anschließend mit einem Teflon/Glas-Potter-Homogenisator (1000 UpM, 3 Stöße) homogenisiert. Der Ansatz wurde anschließend sonifiziert (20 kHz, pulsierend, 5 min, Titanrüssel 3/4"). Solubilisiertes Material wurde durch Zentrifugation entfernt (4 x 50 ml-Becher, 40000 g, 30 min, 0-4°C). Die Überstände wurden verworfen. Die Pellets wurden gut abgetropft, anschließend resuspendiert in 60 ml Glycin-HCl/100 mM/pH3 mit 14 mM Mercaptoethanol und anschließend mit einem Teflon/Glas-Potter-Homogenisator (1000 UpM, 3 Stöße) homogenisiert. Der Ansatz wurde anschließend sonifiziert (20 kHz, pulsierend, 5 min, Titanrüssel 3/4"). Solubilisiertes Material wurde durch Zentrifugation entfernt (2 x 50 ml-Becher, 40000 g, 30 min, 0-4°C). Die Überstände wurden verworfen. Die Pellets wurden gut abgetropft, anschließend resuspendiert in 50 ml Tris-HCl/20 mM/pH7 mit 4 M Harnstoff und 14 mM Mercaptoethanol und mit einem Teflon/Glas-Potter-Homogenisator (1000 UpM, 3 Stöße) homogenisiert. Der Ansatz wurde dann noch sonifiziert (20 kHz, pulsierend, 5 min, Titanrüssel 3/4"). Nichtsolubilisiertes Material wurde durch Zentrifugation entfernt (2 x 50 ml-Becher, 40000 g, 30 min, 0-4°C). Die Sedimente wurden verworfen und die Überstände vereinigt und eingefroren und über Nacht gelagert bei -20° bis -30°C.

Anchließend wurde die solubiliserte Fraktion aufgetaut und es wurde unter Rühren bei Raumtemperatur Harnstoff bis 8 M zugegeben. Diese Proteinlösung wurde über SP-Sepharose (Fast Flow) chromatographiert. Dabei wurde eine Säule der Dimension 2.6 x 12 cm (60 ml) verwendet. A (280 nm) und die Leitfähigkeit wurden kontinuierlich registriert. Der Fluß betrug 5 ml/min. Säulenpuffer war Tris-HCl/20 mM/pH7 mit 8 M Harnstoff und 1.4. mM Mercaptoethanol. Nach Probenauftrag, gefolgt von 100 ml Säulenpuffer, wurde ein flacher linearer NaCl-Gradient (dC/dV = 1.5mM/ml, bis 0.4 M) gefolgt von einem steileren NaCl-Gradient (dC/dV = 6 mM/ml) in Säulenpuffer angelegt. Das Eluat wurde in 10 ml-Fraktionen zerlegt und eingefroren. Die Antigen-enthaltenden Fraktionen fanden sich im Bereich zwischen 150-250 mM NaCl. Der Nachweis erfolgte durch SDS-PA-Disc-Elektrophorese mit anschließender Coomassie-Färbung und Western Blot Analyse.

Die Fraktionen, die serologisch reaktives (Western Blot), intaktes AD1+2-Antigen (27kD Bande) enthielten wurden aufgetaut, vereint und bis zu einem Endvolumen von 5-10 ml mittels Ultrafiltration (Rührzelle, Eisbad, Membran = Omega 30) konzentriert. Das Retentat wurde der Kammer entnommen und über eine Superdex 200 High Load-Säule der Dimension 2.6x60cm chromatographiert. Säulenpuffer war Tris-HCl/20 mM/pH7 mit 8 M Harnstoff und 1.4. mM Mercaptoethanol. A (280 nm) und die Leitfähigkeit wurden kontinuierlich registriert. Der Fluß betrug 5 ml/min. Das Eluat wurde in 10 ml-Fraktionen zerlegt und eingefroren. Die Antigen-enthaltenden Fraktionen fanden sich im späten Fraktionierbereich der Säule. Der Nachweis erfolgte durch SDS-PA-Disc-Elektrophorese mit anschließender Coomassie-Färbung und Western Blot Analyse.

Die Fraktionen, die serologisch reaktives (Western Blot), intaktes AD1+2-Antigen (27kD Bande) enthielten wurden aufgetaut, vereint und über Druckfiltration in einer Rührzelle im Eisbad, (Membran = Omega 30) wiederholt auf halbes Ausgangsvolumen konzentriert und mit Renaturierungspuffer wieder aufgefüllt, sodaß die Harnstoffkonzentration von 8M über 4M, 2M, 1M, 0.5M bis 0.25M reduziert wurde. Die letzte Konzentrierung wurde bis zu einem Endvolumen von 5-10 ml durchgeführt. Renaturierungspuffer war Tris-HCl/100 mM mit 10 % Glycerin und 14 mM Mercaptoethanol. Das Retentat wurde der Kammer entnommen und aliquotiert eingefroren und gelagert bei -60 bis -80°C.

### Beispiel 4

### Reinigung des AD1 deletierten 12k-Fragments des autologen Fusionsproteins AD1+2

Die Anzucht und Expression erfolgte wie im Beispiel 3 für das intakte autologe Fusionsprotein AD1+2 beschrieben.

Die eingefrorenen Bakterienpellets wurden aufgetaut und resuspendiert in 160 ml Basis-Puffer (Tris-HCl/20 mM/pH7.5) und anschließend mit einem (Teflon/Glas-Potter-Homogenisator) homogenisiert. Unter Rühren bei Raumtemperatur wurden folgende Additive zugegeben: NP-40 (0.05 %), PMSF (0.2 mM), Pefabloc (0.2 mM), EDTA (50 mM) und Lysozym (50 mg). Das Gesamtvolumen betrug 200 ml. Der Ansatz wurde nach Lysozymzugabe sofort auf Eis gestellt. Alle weiteren Schritte erfolgten auf Eis bzw. gekühlt. Nach der Inkubation wurde Glycerin (10 %) und 2-Mercaptoethanol (14 mM) zugegeben und vermischt. Das Volumen wurde mit Basis-Puffer auf 280 ml eingestellt. Der Lyseansatz wurde anschließend sonifiziert (20 kHz, pulsierend, 5 min, Titanrüssel 3/4"), gefolgt von maschinellem Pottern (Teflon/Glas-Potter-Homogenisator, 1000 UpM, 10 Stöße). Solubilisiertes Material wurde durch Zentrifugation entfernt (2 x 250 ml-Becher, 15000 g, 10 min, 0-4°C). Die Überstände wurden verworfen.

Die Pellets wurden gut abgetropft und sofort resuspendiert in 100 ml Tris-HCl/100 mM/pH9 mit 10% Glycerin, 0.5% NP-40 und 14 mM Mercaptoethanol und anschließend mit einem Teflon/Glas-Potter-Homogenisator (1000 UpM, 3 Stöße) homogenisiert. Der Ansatz wurde anschließend sonifiziert (20 kHz, pulsierend, 5 min, Titanrüssel 3/4"). Solubilisiertes Material wurde durch Zentrifugation entfernt (4 x 50 ml-Becher, 40000 g, 30 min, 0-4°C). Die Überstände wurden verworfen.

Die Pellets wurden gut abgetropft, anschließend resuspendiert in 60 ml Glycin-HCl/100 mM/pH3 mit 14 mM Mercaptoethanol und anschließend mit einem Teflon/Glas-Potter-Homogenisator (1000 UpM, 3 Stöße) homogenisiert. Der Ansatz wurde anschließend sonifiziert (20 kHz, pulsierend, 5 min, Titanrüssel 3/4"). Solubilisiertes Material wurde durch Zentrifugation entfernt (2 x 50 ml-Becher, 40000 g, 30 min, 0-4°C). Die Überstände wurden verworfen.

Die Pellets wurden gut abgetropft, anschließend resuspendiert in 100 ml Bistris-HCl/20 mM/pH6.5 mit 4 M Harnstoff und 14 mM Mercaptoethanol und mit einem Teflon/Glas-Potter-Homogenisator (1000 UpM, 3 Stöße) homogenisiert. Der Ansatz wurde dann noch sonifiziert (20 kHz, pulsierend, 5 min, Titanrüssel 3/4"). Solubilisiertes Material wurde durch Zentrifugation entfernt (4 x 50 ml-Becher, 40000 g, 30 min, 0-4°C).

Die Pellets wurden gut abgetropft, anschließend resuspendiert in 50 ml Bistris-HCl/20 mM/pH6.5 mit 8 M Harnstoff und 14 mM Mercaptoethanol und mit einem Teflon/Glas-Potter-Homogenisator (1000 UpM, 3 Stöße) homogenisiert. Der Ansatz wurde dann noch sonifiziert (20 kHz, pulsierend, 5 min, Titanrüssel 3/4"). Solubilisiertes Material wurde durch Zentrifugation entfernt (2 x 50 ml-Becher, 40000 g, 30 min, 0-4°C).

Die Pellets wurden gut abgetropft, anschließend resuspendiert in 100 ml Tris-HCl/20 mM/pH9 mit 8 M Harnstoff und 14 mM Mercaptoethanol und mit einem Teflon/Glas-Potter-Homogenisator (1000 UpM, 3 Stöße) homogenisiert. Der Ansatz wurde dann noch sonifiziert (20 kHz, pulsierend, 5 min, Titanrüssel 3/4"). Nicht-solubilisiertes Material wurde durch Zentrifugation entfernt (2 x 50 ml-Becher, 40000 g, 30 min, 0-4°C). Der Überstand wurde eingefroren und über Nacht gelagert bei -20 bis -30°C.

Der Überstand wurde anschließend aufgetaut und über Druckfiltration in einer Rührzelle im Eisbad, (Membran = Omega 30) wiederholt auf halbes Ausgangsvolumen konzentriert und mit Renaturierungspuffer wieder aufgefüllt, sodaß die Harnstoffkonzentration von 8M über 4M, 2M, 1M, 0.5M bis 0.25M reduziert wurde. Die letzte Konzentrierung wurde bis zu einem Endvolumen von ca. 5 ml durchgeführt. Renaturierungspuffer war Tris-HCl/100 mM mit 10 % Glycerin und 14 mM Mercaptoethanol. Während des Harnstoff-Entzugs präzipitierte das Protein und wurde nach Abzentrifugieren mit 0.2% SDS in Renaturierungspuffer resuspendiert. Diese Lösung, die hochreines 12k-Fragment enthielt, wurde aliquotiert eingefroren und gelagert bei -60 bis -80°C.

### Beispiel 5:

### Western Blot Test

Es wurden verschiedene gB-Antigene als ungereinigte Lysatproben in Western Blot-Experimenten eingesetzt und die Ergebnisse wurden miteinander verglichen. Die hierbei erhaltenen Ergebnisse sind in Tabelle 3 und 4 zusammengefaßt.

**Tabelle 3**

| **Reaktivität der verschiedenen rekombinanten gB-Antigene im IgG-Western Blot** | | | | | |
|---|---|---|---|---|---|
| **Vergleich AD1 und AD2 aus AD169** | | | | | |
| Serum/Antigen | AD1-GST | AD2-GST | AD1+2 [27k] | AD1+2 [12k] | GST-Kontrolle |
| Suhl 33 | - | - | - | - | - |
| Suhl 73 | - | +++ | + | +++ | - |
| Suhl 81 | - | - | - | - | - |
| Suhl 120 | - | +++ | + | ++ | - |
| DRK 98 | (+) | +++ | + | +++ | - |
| DRK 129 | + | +++ | + | +++ | - |
| DRK 136 | - | ++ | (+) | + | - |
| DRK 216 | + | +++ | ++ | ++ | - |

### Erklärung der Tabelle 3 HCMV-gB-Patent WB

Die Tabelle zeigt die Ergebnisse von Western Blot Experimenten (WB):

Eingesetzte Antigene waren: AD1 und AD2 als GST Proteine, zusammen mit GST Kontrolle (gereinigte Glutathion S-Transferase) sowie direkt exprimiertes Fusionsprotein AD1+2. Dieser Klon exprimierte intaktes AD1+2 (=27k-Bande) nur sehr schwach, ein kleineres Fragment jedoch sehr stark (=12k-Bande). Im Western Blot wurden ungereinigte Rohlysate (AD1- und AD2-GST) teilgereinigte Fraktionen (AD1+2,27k+12k) bzw. gereinigtes Protein (GST) eingesezt.

Als Seren wurden zufällige, HCMV-positive Blutspender (n=8) eingesetzt. Der Nachweis erfolgte auf IgG Antikörper.

### Ergebnisse:

Diese Tabelle soll folgendes zeigen:
1. AD2 ist viel reaktiver als AD1. AD1 hat nur eine geringe, nicht ausreichende Sensitivität (trotz gleicher Mengen im Blot).
2. Die intakte Bande von AD1+2 (27k) und das Fragment (12k) reagieren übereinstimmend. Die stärkere Reaktion des Fragments erklärt sich hier durch eine viel größere Antigenmenge im Blot.
3. Auch AD2-GST und AD1+227k bzw. 12k zeigen volle Übereinstimmung.

### Analyse des 12k Fragments von AD1+2:

Das Fragment von AD1+2 konnte leicht abgetrennt und homogen gereingt werden. Eine N-terminale Ansequenzierung ergab die Sequenz des N-Terminus von AD1+2. Auf Grund der Größe enthält das Fragment vollständig den AD2-Bereich und höchstens einige wenige Aminosäuren am Anfang des AD1.

### Schlußfolgerung:

Die Western Blot Daten zeigten früh, daß AD2 einen hoch reaktiven Bereich darstellt und AD1 weder als GST-Protein, noch als Bestandteil des AD1+2 eine verwertbare serologische Reaktion besitzt. Diese überraschende Ergebnis konnte auch mit synthetischen Peptiden im ELISA bestätigt werden.

**Tabelle 4**

| **Reaktivität der verschiedenen rekombinanten AD2-Antigene im IgG-Western Blot** | | | | | |
|---|---|---|---|---|---|
| **Vergleich AD2 aus AD169 mit AD2 aus Towne** | | | | | |
| Strain | AD169 | Towne | AD+Towne | AD+Towne | Kontrolle |
| Serum/Antigen | AD2-GST | AD2-GST | AD2+AD2-GST | AD2+AD2 | GST |
| Suhl 81 | - | - | - | - | - |
| Suhl 120 | +++ | - | + | + | - |
| DRK 88 | ++ | +++ | +++ | ++ | - |
| DRK 98 | +++ | ++ | + | + | - |
| DRK 129 | +++ | +++ | ++ | ++ | - |
| DRK 145 | - | - | - | - | - |
| DRK 240 | - | - | - | - | - |
| DRK 313 | - | + | + | + | - |

### Erklärung der Tabelle 4 HCMV-gB-Patent WB

Die Tabelle 4 zeigt die Ergebnisse von Western Blot Experimenten:

Eingesetzte Antigene waren: AD2 aus AD169, AD2 aus Towne, beide als GST Proteine, sowie die autologe Fusion der beiden Sequenzen als direkt exprimiertes Protein und als GST-Fusionsprotein. Außerdem noch die GST Kontrolle (gereinigte Glutathion S-Transferase).

Im Western Blot wurden ungereinigte Rohlysate bzw. gereinigtes Protein (GST) eingesezt.

Als Seren wurden zufällige, HCMV-positive Blutspender (n=8) eingesetzt. Der Nachweis erfolgte auf IgG Antikörper.

### Ergebnisse:

Die Tabelle 4 soll folgendes zeigen:
1. AD2 aus Towne hatte eine vergleichbare Reaktivität wie AD2 aus AD169.
2. Es gibt stammspezifische Seren, sowohl für die AD169 Sequenz (Suhl 120) als auch für die Towne Sequenz (DRK313), die nicht kreuzreagieren.
3. Die autologen Fusionen AD2/AD169 mit AD2/Towne sind technisch machbar, d.h. werden stabil exprimiert und zeigten eine additive Reaktivität. Die Kombination der beiden Sequenzen war sensitiver, als jede Einzelkomponente.

Ausgehend von diesen WB Experimenten wurden folgende Antigene für ELISA Experimente gereinigt:
AD2-GST, AD1+2(27k) und AD1+2 (12k).

Zusätzlich wurden AD1, AD2/AD169 und AD2/Towne (verkürzt) chemisch synthetisiert und im ELISA evaluiert.

### Beispiel 6

### Reaktivität der gB Komponenten im ELISA

Um die einzelnen antigenen Domänen von gB des Stammes AD169 auf ihre Reaktivität hin zu untersuchen, wurden ELISA-Analysen mit Seren von gesunden Blutspendern durchgeführt. Dazu wurden die antigenen Domänen 1 und 2 (AD1 und AD2) als rekombinantes Fusionsprotein prokaryot in dem Expressionsvektor pET5c exprimiert und gereinigt. Das Fusionsprotein umfaßte im N-terminalen Vorderteil die AS 24-87 von AD2 und im C-terminalen Abschnitt die AS 545-679 von AD1. Zur genaueren Differenzierung der Einzelkomponenten wurde sowohl AD1 als auch AD2 als Peptide synthetisch hergestellt. Das AD1-Peptid bestand aus den AS 549-630 und das AD2-Peptid enthielt die AS 27-84. Zusätzlich wurde AD2 (AS 27-84) als rekombinantes Gluthathion-S-Transferase (GST) Fusionsprotein in *E.coli* separat exprimiert und gereinigt.

Je 1 µg der gereinigten rekombinanten Antigene AD1+2 und GST-AD2 und der synthetischen Peptide AD1 und AD2 gelöst in 100 µl 0,01 M Carbonatpuffer pH 9,5, wurden jeweils in die Vertiefung von Mikrotiterplatten (Nunc, Maxisorp) gegeben und 16 h in einer geerdeten, feuchten Kammer inkubiert. Nach Zugabe von 100 µl einer Kalbsserum haltigen Nachbeschichtungslösung, wurde die Inkubation für 2 weitere Stunden fortgesetzt. Danach wurden die Platten entleert und sorgfältig ausgeschlagen. Anschließend wurden die beschichteten Platten für die eigentliche Testung verwendet, oder nach Trocknung im Vakuumschrank, mit anschließendem Einschweißen in einem Folienschlauch bis zum späteren Gebrauch bei -20°C gelagert. Für die Testdurchführung wurden die Testnäpfchen der ELISA-Platten mit 100 µl einer 1:21 verdünnten Serumlösung gefüllt, mit einer Plastikfolie abgeklebt und bei 37°C im Brutschrank (Binder, BED53) für 1 h inkubiert. Nach dreimaligem Waschen im Biotest Washer II erfolgte die Konjugatinkubation mit 100 µl von gegen humanes IgG gerichteten, mit Peroxidase-markierten, monoklonalen Antikörpern aus der Maus (30 min bei 37°). Nach abermaligem Waschen wurden die gebundenen Antikörper durch die Enzymaktivität der Peroxidase mittels 100 µl 3,3',5,5'-Tetramethylbenzidine (TMB, Sigma) sichtbar gemacht. Die Chromogenreaktion dauerte 30 min bei Raumtemperatur und wurde durch die Zugabe von 100 µl 1 N Schwefelsäure beendet. Die optische Dichte (OD) der einzelnen Proben wurde bei 495 nm (Referenz: 620 nm) im Anthos HTII ELISA-Reader ermittelt. Alle OD-Werte größer als 300 mOD wurden als positiv bewertet. Die Ergebnisse sind in Tabelle 5 zusammengefaßt.

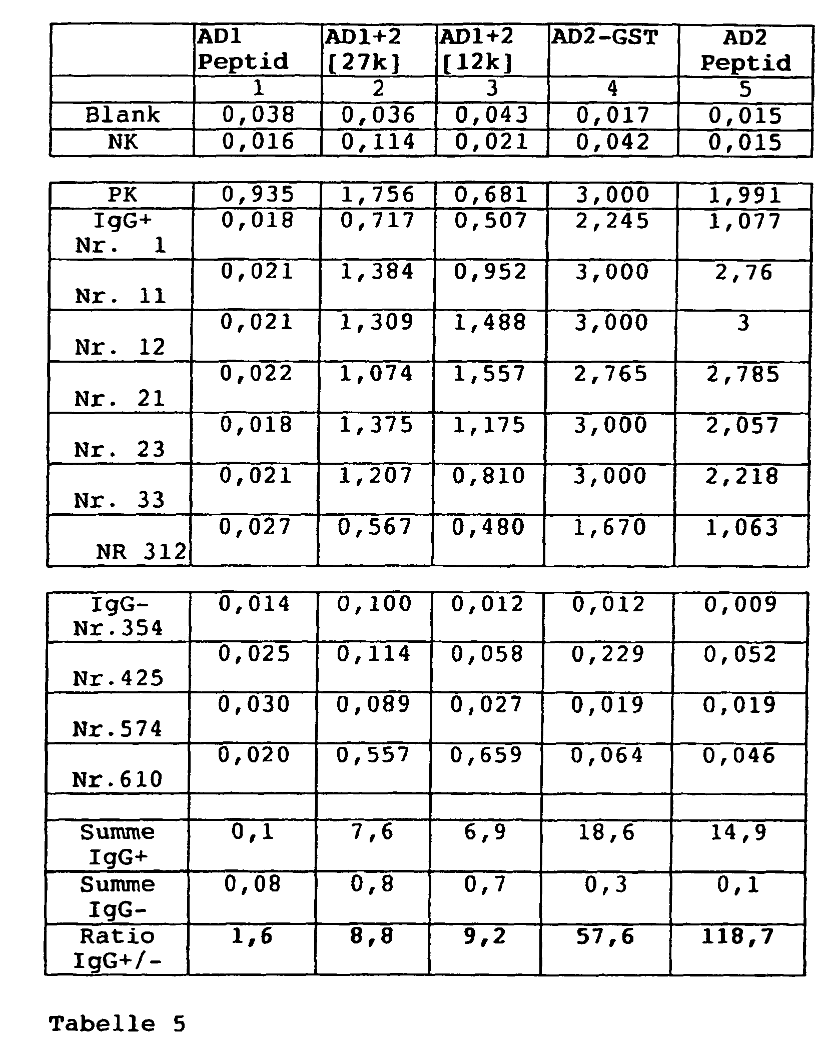

In der Tabelle 5 sind die ELISA-Ergebnisse zusammengefaßt. Mit dem AD1-Peptid (gB549-630) konnten keine erhöhten OD-Werte gegenüber dem Null-Wert (Blank) beobachtet werden. Eine Erklärung dafür könnte sein, daß die Konformation von AD1, die für die immunologische Reaktivität notwendig ist, im synthetischen Peptid nicht ausgeprägt ist oder durch technische Begebenheiten bei der Beschichtung (z.B. Pufferbedingungen) nicht funktionell vorliegt. Das rekombinante Fusionsprotein AD1+2 [27k] ist reaktiver als das AD1-Peptid und zeigt ein ähnliches Reaktionsprofil wie AD1+2 [12k], welches im wesentlichen den N-terminalen Bereich von gB (AD-2) umfaßt. Diese Ergebnisse lassen den Schluß zu, daß die immunologische Reaktivität von AD1+2 durch AD2 und nicht durch AD1 vermittelt wird. Die Ergebnisse lassen vermuten, daß AD1 weder als synthetisches Peptid noch als rekombinantes Fusionsprotein im ELISA einen essentiellen Beitrag leistet. Die besten Ergebnisse zeigten das Fusionsprotein AD2-GST und das AD2-Peptid, wobei die Trennung zwischen positiven und negativen Seren im Falle des Peptids von AD2 noch ausgeprägter war als mit dem rekombinanten Fusionsprotein AD2-GST.

Diese Daten belegen, daß ein kleines Polypeptid, welches den N-terminalen Teil von gB im Bereich von AS 27-84 umfaßt, überraschende antigene Eigenschaften besitzt, die vorteilhafterweise in einem diagnostischen gB ELISA verwendet werden können.

### Beispiel 7

### Entwicklung eines HCMV-gB-IgG ELISA mit AD2-Peptiden von zwei HCMV-Stämmen

### AD2-Peptid-IgG-ELISA

Es wurde ein AD2-Peptid entworfen und synthetisiert, die der Aminosäuresequenz zwischen Position 27 und 84 aus gB vom HCMV-Stamm AD169 entspricht. Für dieses Peptid AD2 wurde zunächst in Vorversuchen die optimale Beschichtungskonzentration und Konjugateinstellung ermittelt. Ausgehend von 160 HCMV-IgG-negativen Spenderseren wurde bei einer Plattenbeschichtung von 0,1 µg/ml AD2-Peptid und einer Konjugateinstellung von ca. 5 µl BS-12 (anti-human-IgG-Fc) der cutoff dieses Testes zu ca. 0,300 definiert. Bei diesen Bedingungen waren von den 160 IgG-negativen Seren 159 testnegativ, dies entspricht einer analytischen Spezifität von 99,4 %.

Mit dem AD2-Peptid von AD169 zeigte der ELISA zwar eine hervorragende Spezifität, die Sensitivität bei sekundären HCMV-Infektionen lag allerdings nur bei 77 %. Daher wurde erfindungsgemäß ein AD2-homologes Peptid aus dem HCMV-Stamm Towne zusätzlich zu AD2 von AD169 verwendet, um neutralisierenden Antikörpern im Serum von HCMV-IgG-Positiven zu erfassen, die nicht mit AD2 von AD169 kreuzreagierten. Für dieses Peptid (=AS 27-73 von gB Towne) wurde zunächst in Vorversuchen die optimale Beschichtungskonzentration und Konjugateinstellung ermittelt. Ausgehend von 160 HCMV-IgG-negativen Spenderseren wurde bei einer Plattenbeschichtung von 0,7 µg/ml AD2/Towne-Peptid und einer Konjugateinstellung von ca. 15 µl BS-12 (anti-human-IgG-Fc) der cutoff dieses Testes zu ca. 0,500 definiert. Bei diesen Bedingungen waren von den 160 IgG-negativen Seren 157 testnegativ, dies entspricht einer analytischen Spezifität von 98,8 %. Auch die Kombinationsfestphase aus AD2-Peptidgemisch wurde mit den 160 HCMV-IgG-negativen Spendern untersucht: bei einem cutoff von 0,200 OD fand sich kein positives Serum, die Spezifität beträgt für den Antigenmix unter diesen Bedingungen demnach sogar 100 %.

161 HCMV-IgG-positive Spenderseren von gesunden Blutspendern wurden sowohl mit den AD2-Einzelantigen ELISAs als auch mit dem kombinierten AD2-Mix ELISA untersucht. Die unterschiedlichen Reaktionen mit den AD2-Peptiden widerspiegelt vermutlich die Verteilung von AD169-ähnlichen, Towne-ähnlichen und kreuzreaktiven HCMV-Stämmen in den hier untersuchten Humanpopulationen. In den Einzelantigen-ELISAs sprachen von 161 Proben 23 Seren gleichzeitig auf AD2 aus AD169 und Towne an (=14,3 %). Diese Seren sind kreuzreaktiv für beide HCMV-Stämme. Monospezifisch für AD2 waren 69 Proben (=42.9 %). Sie reagierten nur mit AD2 aus AD169 und nicht mit AD2 aus Towne. Insgesamt 15 Proben sprachen nur auf die Towne-Sequenz an (=9,3 %). Bei der unterschiedlichen Sensitivität der AD2-ELISA muß berücksichtigt werden, daß die Towne-Sequenz des synthetischen Peptids um 11 Aminosäuren kürzer war als die AD2-Sequenz am AD169. Die restlichen 54 Proben zeigten keine Reaktion gegenüber beiden Antigenen (=33,5 %). Das AD2-Gemisch erfaßt 112 HCMV IgG positive Seren von 161 Proben (=69,6 %) und vermittelt eine höhere Sensitivität als die jeweiligen Einzelkomponenten ELISA's. Alle Seren, die in den Einzeltests detektiert werden, sind auch im Misch-ELISA positiv. Die Ergebnisse mit den Seren von HCMV positiven Blutspendern bestätigen, daß nur das Gemisch der beiden gB Peptide von AD169 und Towne eine höhere Sensitivität vermittelt als die jeweiligen Tests der Einzelkomponenten.

Die Tabellen 6 a)-c) zeigen die Ergebnisse der Peptid-ELISAs:

### Sensitivitäten der AD2-Peptid-IgG ELISAs in HCMV-seronegativen und -seropositiven Blutspendern:

### • Ergebnisse für N=160 HCMV-seronegative Blutspender, Spezifitätsbestimmung:

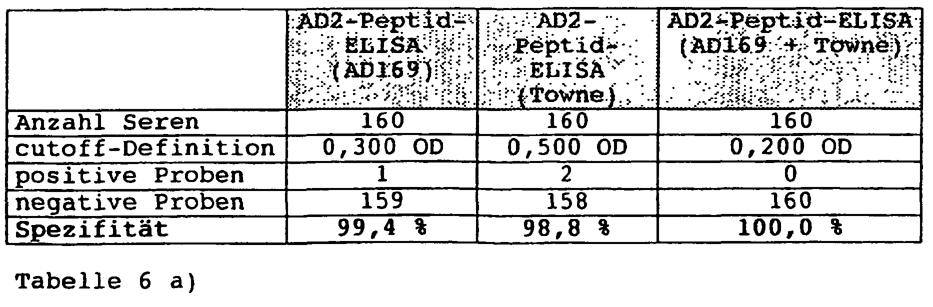

### AD2-Peptid-ELISA: Vergleich AD169 mit Towne

### • Ergebnisse für N=161 HCMV-seropositive Blutspender:

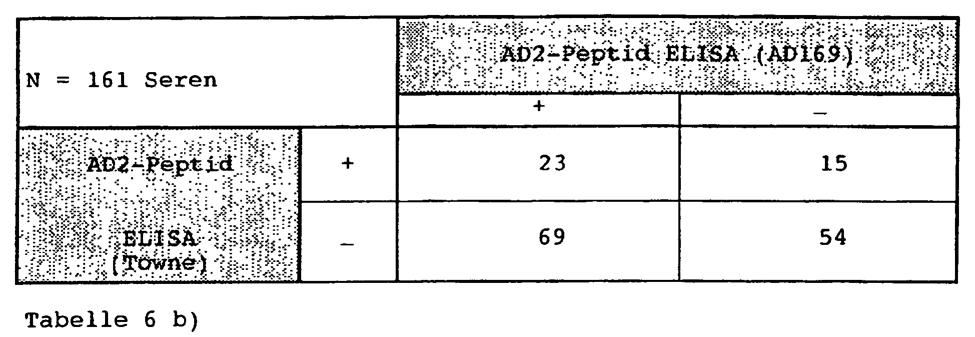

### AD2-Kombinations-ELISA: AD169 + Towne

### • Ergebnisse für N=161 HCMV-seropositive Blutspender:

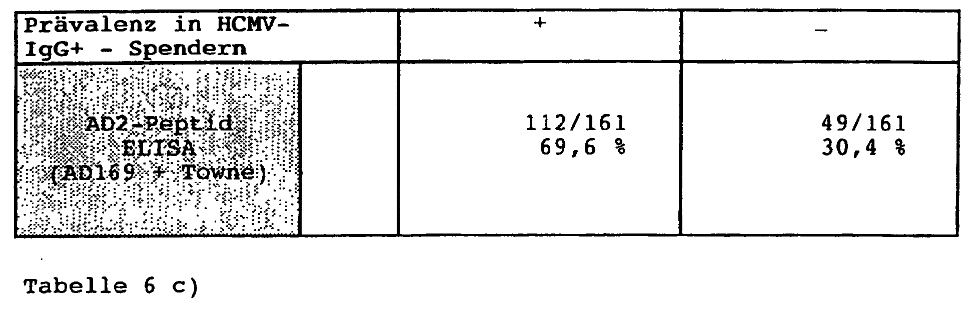

### Beispiel 8

### Ergebnisse bei Transplantationspatienten

Getestet wurden 7 HCMV-Primärinfektionsverläufe und 22 HCMV-Sekundärinfektionsverläufe aus der Transplantationsroutine.

Entsprechend dem Literaturkenntnisstand werden bei primären HCMV-Verlaufsseren erst nach ca. 60-80 Tagen post infectionem, bei sekundären HCMV-Verlaufsseren dagegen sofort nach Reaktivierung bzw. Reinfektion neutralisierende Antikörper gebildet. Um dies zu überprüfen, wurden 7 HCMV-Primärinfektionsverläufe und 22 HCMV-Sekundärinfektionsverläufe aus der Transplantationsroutine untersucht. Bei Betrachtung der primären HCMV-Verlaufsseren bis ungefähr zum Tag 80 post infectionem wurden von 7 Verläufen (=7 Patienten) keine positiven Proben gefunden. Bei den sekundären HCMV-Verläufen (N=22, 22 Patienten) waren 17 Patienten durchgehend positiv für neutralisierende Antikörper gegen das AD2-Peptid des AD169, 5 Verläufe blieben negativ. Demnach beträgt die Spezifität 100 % und die analytische Sensitivität 77 % in diesem Kollektiv.

Durch den zusätzlichen Einsatz von AD2 aus Towne als Peptid (0,7 µg/ml) zu dem AD2-Peptid aus AD169 (0,2 µg/ml) kam es zu keiner Spezifitätsveränderung (7 von 7 HCMV-Primärinfektionsverläufen negativ, Spezifität=100 %). Bei den HCMV-Sekundärinfektionen sprachen jedoch 2 zusätzliche Verläufe auf das Towne-spezifische Antigen an, die mit AD2 von AD169 negativ waren. So wurde die Gesamtsensitivität von 77% für AD2 durch die Zugabe von TW2 auf 86% gesteigert. Die Kombination von AD2-AD169 und -Towne vermittelte somit einen Gewinn von 9 %.

Die Ergebnisse sind in Tabelle 7 a)-c) zusammengefaßt.

### Sensitivität im AD2-IgG (AD169 + Towne)-ELISA in HCMV-Primär-und Sekundärinfektionen:

- Kollektive: 1.) 7 HCMV-Primärinfektionsverläufe (nach Tx)
   2.) 22 HCMV-Sekundärinfektionsverläufe (nach Tx)
- Kategorisierung:

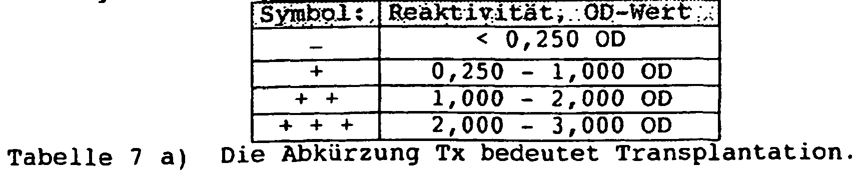

### Ergebnisse, HCMV-Primärinfektionen (N=7), Beurteilung bis Tag 80 post-Tx:

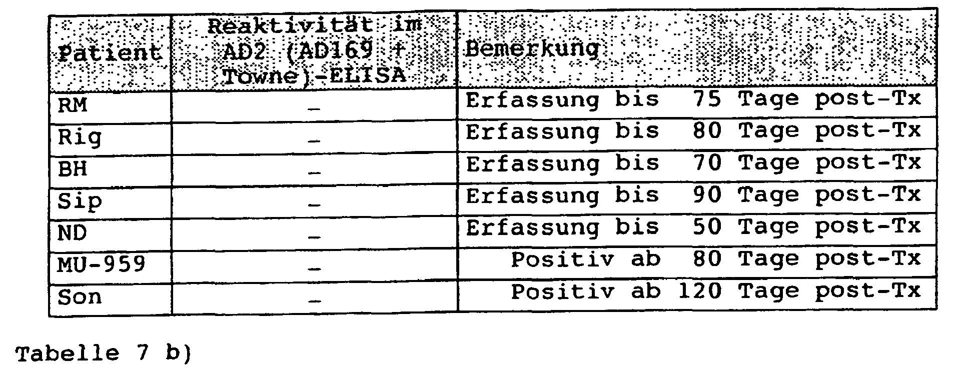

### Ergebnisse, HCMV-Sekundärinfektionen (N=22), Beurteilung bis Tag 80 post-Tx:

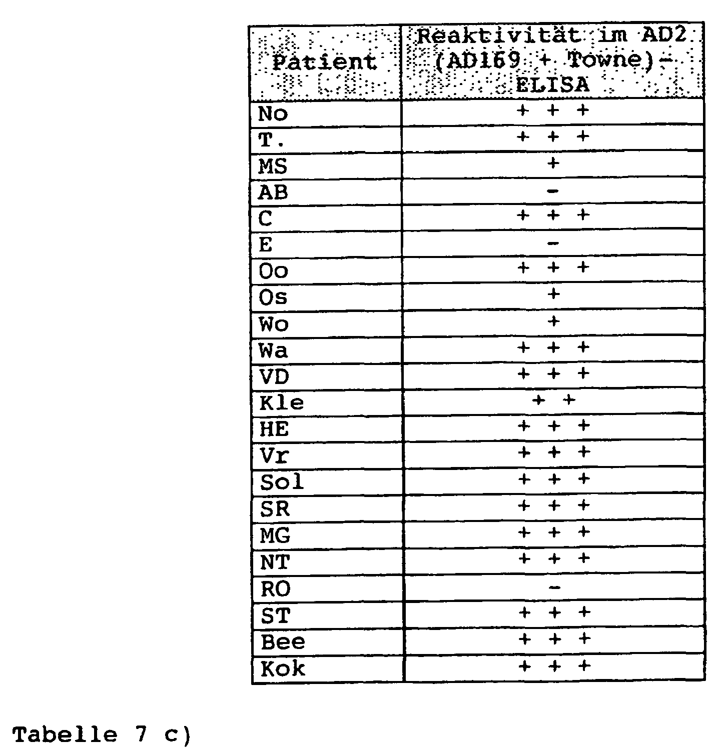

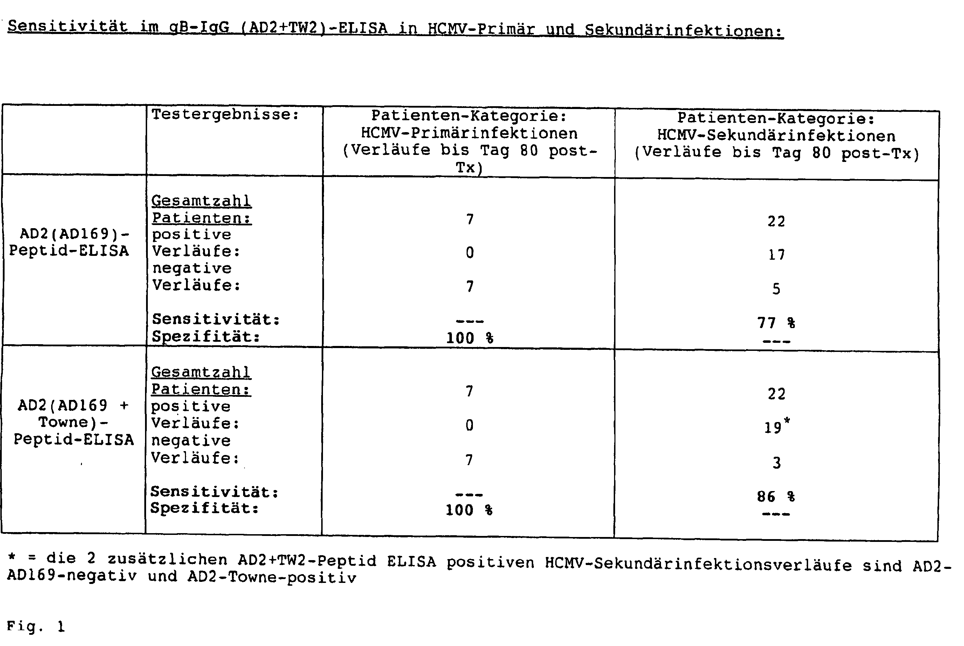

Eine Gesamtzusammenfassung der Ergebnisse findet sich in Fig. 1.

### Beispiel 9

### Ergebnisse bei HIV-positiven Patienten

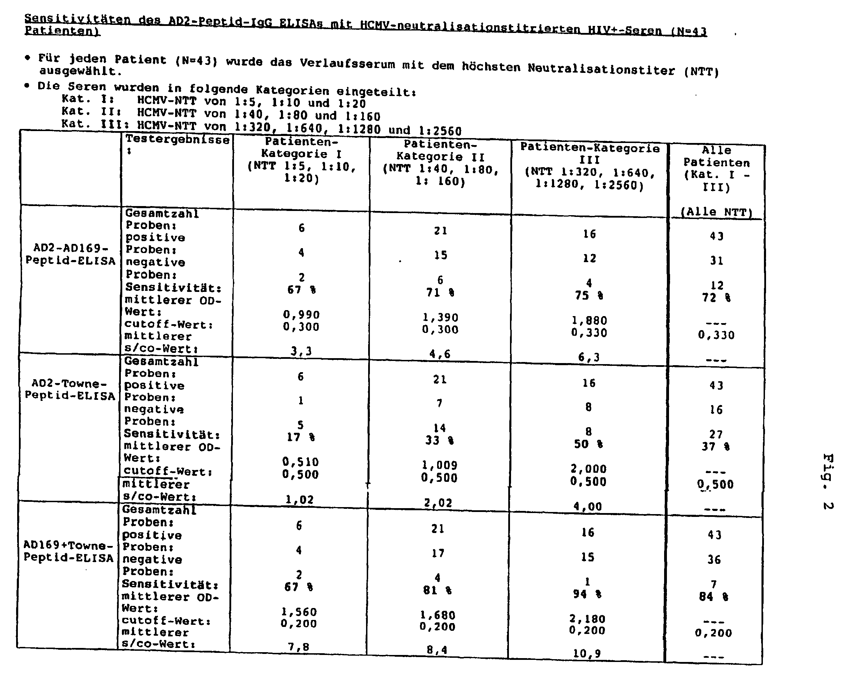

43 neutralisationstitrierte Seren von HIV-Positiven (Titration im Bioassay) wurden überprüft. Die Ergebnisse sind in Fig. 2 zusammengefaßt.

Bei HIV-Positiven bzw. AIDS-Erkrankten beträgt die HCMV-Prävalenz nahezu 100 %. Die vorliegenden Seren wurden in einem Zellkultur-Neutralisationsassay (Bioassay) austitriert. Um zu überprüfen, ob ein hoher HCMV-Neutralisationstiter mit einem entsprechend hohen positiven Signal im AD2-Peptid-ELISA korreliert, wurden 3 Patientengruppen gebildet:

Kategorie I enthält Seren (N=6) mit Neutralisationstitern von 1:5 bis 1:20,

Kategorie II (N=21) enthält Seren mit Neutralisationstitern von 1:40 bis 1: 160 und

Kategorie III (N=16) enthält Seren mit Neutralisationstitern von 1:320 bis 1:2560.

Jede Serumprobe entspricht einem untersuchten Patienten, es wurde pro Patient die jeweils hochtitrigste Probe ausgewählt. In Kategorie I waren 4 von 6 Seren positiv (Sensitivität=67 %), in Kategorie II 15 von 21 Seren positiv (Sensitivität=71 %) und in Kategorie III waren 12 von 16 Proben positiv (Sensitivität= 75 %). Die Höhe des Neutralisationstiters aus dem Bioassay korreliert mit der Reaktivität (mittlerer, arithmetischer OD-Wert) im AD2/AD169-ELISA: Kategorie I: OD=0,990, Kategorie II: OD=1,390 und Kategorie III: OD=1,880).

Durch den kombinierten Einsatz der AD2-Peptide aus AD169 und Towne als Festphasenantigene wurden auch in diesem Kollektiv klare Sensitivitätsgewinne erzielt: In Kategorie I waren nach wie vor 4 von 6 Seren positiv (Sensitivität=67 %). In Kategorie II waren hingegen 17 von 21 Seren positiv (Sensitivität=81 %, zuvor: 71 %) und in Kategorie III waren insgesamt 15 von 16 Proben positiv (Sensitivität=94 %, zuvor: 75 %). Die Höhe des Neutralisationstiters aus dem Bioassay korrelierte mit der Reaktivität (mittlerer, arithmetischer OD-Wert) im AD2-Misch-ELISA: Kategorie I: OD=1,560, Kategorie II: OD=1,680 und Kategorie III: OD=2,180). Faßt man alle Patienten-Kategorien (I, II und III) zusammen ergibt sich für AD2 eine Sensitivität von 72 %. Durch die Ergänzung von AD2 aus AD169 mit dem Towne-spezifischen, homologen Antigen steigt die Sensitivität in allen drei Patientenkategorien um 12 % auf 84 %. Der Sensitivitätsgewinn durch das AD2-Peptid aus Towne beträgt im Mittel also ca. 10 % für beide Sensitivitätskollektive (HCMV-Sekundärinfektionen und HIV/ HCMV-Positive).

### Beispiel 10:

### Untersuchung von Immunglobulinpräparaten

Zwei kommerziell erhältliche Immunglobulinpräparate wurden über mehrere Titerstufen austitriert und ihre Reaktivität im gB-ELISA getestet (siehe Tabelle 8). Bei dem Präparat A handelt es sich um polyvalentes humanes Immunglobulin, während das Präparat B ein anti-HCMV Hyper-Immunglobulin darstellt. Präparat A zeigte bis zu einer Titerstufe von 1:168 positive Ergebnisse im gB-ELISA. Mit Präparat B konnte bis zu einer Titerstufe von 1:2688 noch ein positives Signal im ELISA detektiert werden. Es ergab sich ein Unterschied in der Konzentration von HCMV gB spezifischen Antikörpern von vier Titerstufen. Die Konzentration von AD2-spezifischen und potentiell neutralisierenden Antikörpern war im Präparat B um das 16-fache höher als im Präparat A.

**Tabelle 8**

| Titerstufen | Immunglobulin Präparat A | Immunglobulin Präparat B |
|---|---|---|
| 1:42 | 1,263 | 3,0 |
| 1:84 | 0,651 | 3,0 |
| 1:168 | 0,292 | 2.404 |
| 1:336 | 0,153 | 1,551 |
| 1:672 | 0,089 | 0,862 |
| 1:1344 | 0,065 | 0,472 |
| 1:2688 | 0,042 | 0,250 |

## Patentansprüche

1. Polypeptid mit humanem Cytomegalievirus (HCMV) spezifischen Sequenzen, das wenigstens zwei Polypeptidfragmente umfaßt, wobei jedes Fragment aus einander entsprechenden Regionen aus dem N-terminalen Bereich des Glykoproteins gp116 eines HCMV-Stammes herstammt, **dadurch gekennzeichnet, dass** es sich bei dem N-terminalen Bereich des Glykoproteins gp116 um den AD2-Bereich handelt, wobei die AD2-Region des einen Polypeptidfragments von dem Cytomegalievirus-Stamm mit der Bezeichnung AD169 herstammt und die AD2-Region des anderen Polypeptidfragments von dem Cytomegalievirus-Stamm mit der Bezeichnung Towne herstammt.

2. Polypeptid nach Anspruch 1, **dadurch gekennzeichnet, daß** die AD2-Region den Bereich zwischen den Aminosäuren 27 und 84 des Stammes AD169 umfaßt.

3. Polypeptid nach Anspruch 2, **dadurch gekennzeichnet, daß** die AD2-Region von AD169 die Aminosäuresequenz gemäß Sequenz-ID-Nr. 1 umfaßt.

4. Polypeptid nach Anspruch 1, **dadurch gekennzeichnet, daß** die AD2-Region den Bereich zwischen den Aminosäuren 27 und 88, insbesondere zwischen den Aminosäuren 27 und 73, des Cytomegalievirus-Stamms Towne umfasst.

5. Polypeptid nach Anspruch 4, **dadurch gekennzeichnet, daß** die AD2-Region die Aminosäuresequenz mit der Sequenz-ID-Nr. 2 umfaßt.

6. Polypeptid nach einem der vorhergehenden Ansprüche, **dadurch gekennzeichnet, daß** die wenigstens zwei homologen Regionen aus dem N-terminalen Bereich des Glykoproteins gp116 auf unterschiedlichen Polypeptidfragmenten lokalisiert sind, die entweder in Form einer Mischung oder in kovalent verbundener Form vorliegen.

7. Polypeptid nach Anspruch 6, **dadurch gekennzeichnet, daß** das Polypeptid mittels einer chemischen Synthese hergestellt wurde.

8. Polypeptid nach Anspruch 6, **dadurch gekennzeichnet, daß** das Polypeptid rekombinant hergestellt wurde.

9. Polypeptid nach Anspruch 8, **dadurch gekennzeichnet, daß** das Polypeptidfragment in Form eines heterologen Fusionsproteins vorliegt.

10. Polypeptid nach Anspruch 8, **dadurch gekennzeichnet, daß** das Polypeptid als autologes Fusionsprotein vorliegt.

11. Polypeptid nach einem der Ansprüche 6-10, **dadurch gekennzeichnet, daß** sich die Polypeptidfragmente mit den Aminosäuresequenzen entsprechend den homologen Regionen aus dem N-terminalen Bereich von gp116 auf einer Polypeptidkette befinden.

12. Polypeptid nach einem der Ansprüche 6-10, **dadurch gekennzeichnet, daß** die einzelnen Polypeptidfragmente durch chemische Koppelung miteinander verbunden sind.

13. Testkit zum Nachweis von Antikörpern gegen HCMV, **dadurch gekennzeichnet, daß** es ein Polypeptid nach einem der Ansprüche 1-12 umfaßt.

14. Testkit nach Anspruch 13, **dadurch gekennzeichnet, daß** es zum Nachweis von schützenden Antikörpern gegen HCMV geeignet ist.

15. Testkit nach einem der Ansprüche 13 oder 14, **dadurch gekennzeichnet, daß** es ein Testkit zur Durchführung eines ELISA-Test ist.

16. Verwendung eines Polypeptids nach einem der Ansprüche 1-12 in der Diagnostik.

17. Verwendung nach Anspruch 16, **dadurch gekennzeichnet, dass** ein Polypeptid nach einem der Ansprüche 1-12 zum Screenen von humanem Plasma oder zur Auswahl von Plasmaspenden für die Herstellung von HCMV-Hyperimmunglobulin verwendet wird.

18. Impfstoff zur Prophylaxe gegen eine HCMV-Infektion, **dadurch gekennzeichnet, daß** der Impfstoff ein Polypeptid nach einem der Ansprüche 1-12 umfaßt.

19. Verwendung eines Polypeptids nach einem der Ansprüche 1-12 zur Herstellung eines Impfstoffs, der prophylaktisch gegen HCMV-Infektionen wirkt.

## Claims

1. Polypeptide having sequences specific to human cytomegalovirus (HCMV), comprising at least two polypeptide fragments, each fragment originating from regions of the N-terminal region of the glycoprotein gp116 of an HCMV strain, which correspond to each other, **characterized in that** the N-terminal region of the glycoprotein gp116 is the AD2 region, wherein the AD2 region of one polypeptide fragment originates from the cytomegalovirus strain having the designation AD169 and the AD2 region of the other polypeptide fragment originates from the cytomegalovirus strain having the designation Towne.

2. Polypeptide according to claim 1, **characterized in that** the AD2 region comprises the region between the amino acids 27 and 84 of the strain AD169.

3. Polypeptide according to claim 2, **characterized in that** the AD2 region of AD169 comprises the amino acid sequence according to sequence ID No. 1.

4. Polypeptide according to claim 1, **characterized in that** the AD2 region comprises the region between the amino acids 27 and 88, in particular between the amino acids 27 and 73, of the cytomegalovirus strain Towne.

5. Polypeptide according to claim 4, **characterized in that** the AD2 region comprises the amino acid sequence having the sequence ID No. 2.

6. Polypeptide according to one of the preceding claims, **characterized in that** the at least two homologous regions from the N-terminal region of the glycoprotein gp116 are localized in different polypeptide fragments which are present either in the form of a mixture or in covalently bonded form.

7. Polypeptide according to claim 6, **characterized in that** the polypeptide was prepared by means of a chemical synthesis.

8. Polypeptide according to claim 6, **characterized in that** the polypeptide was prepared by recombinant means.

9. Polypeptide according to claim 8, **characterized in that** the polypeptide fragment is present in the form of a heterologous fusion protein.

10. Polypeptide according to claim 8, **characterized in that** the polypeptide is present as an autologous fusion protein.

11. Polypeptide according to one of claims 6-10, **characterized in that** the polypeptide fragments having the amino acid sequences corresponding to the homologous regions from the N-terminal region of gp116 are located on a polypeptide chain.

12. Polypeptide according to one of claims 6-10, **characterized in that** the individual polypeptide fragments are bonded to one another by chemical coupling.

13. Test kit for the detection of antibodies against HCMV, **characterized in that** it comprises a polypeptide according to one of claims 1-12.

14. Test kit according to claim 13, **characterized in that** it is suitable for the detection of protective antibodies against HCMV.

15. Test kit according to one of claims 13 or 14, **characterized in that** it is a test kit for carrying out an ELISA test.

16. Use of a polypeptide according to one of claims 1-12 in diagnosis.

17. Use according to claim 16, **characterized in that** a polypeptide according to one of claims 1-12 is used for the screening of human plasma or for the selection of plasma donors for the production of HCMV hyperimmune globulin.

18. Vaccine for prophylaxis against an HCMV infection, **characterized in that** the vaccine comprises a polypeptide according to one of claims 1-12.

19. Use of a polypeptide according to one of claims 1-12 for the production of a vaccine which acts prophylactically against HCMV infections.

## Revendications

1. Polypeptide avec des séquences spécifiques au cytomégalovirus humain (HCMV), qui comprend au moins deux fragments polypeptidiques, chaque fragment provenant de régions correspondant réciproquement au domaine N-terminal de la glycoprotéine gp116 d'une souche de HCMV, **caractérisé en ce que** le domaine N-terminal de la glycoprotéine gp116 est un domaine AD2, la région AD2 de l'un des fragments polypeptidiques provenant de la souche du cytomégalovirus ayant la désignation AD169 et la région AD2 de l'autre fragment polypeptidique provenant de la souche du cytomégalovirus ayant la désignation Towne.

2. Polypeptide selon la revendication 1, **caractérisé en ce que** la région AD2 comprend le domaine entre les acides aminés 27 et 84 de la souche AD 169.

3. Polypeptide selon la revendication 2, **caractérisé en ce que** la région AD2 de AD 169 comprend la séquence d'acides aminés selon la séquence ID n°1.

4. Polypeptide selon la revendication 1, **caractérisé en ce que** la région AD2 comprend le domaine entre les acides aminés 27 et 88, en particulier entre les acides aminés 27 et 73, de la souche du cytomégalovirus Towne.

5. Polypeptide selon la revendication 4, **caractérisé en ce que** la région AD2 comprend la séquence d'acides aminés ayant la séquence ID n°2.

6. Polypeptide selon l'une des revendications précédentes, **caractérisé en ce que** les au moins deux régions homologues constituées du domaine N-terminal de la glycoprotéine gp116 sont localisés sur différents fragments polypeptidiques , qui se présentent soit sous forme d'un mélange soit sous forme liée de manière covalente.

7. Polypeptide selon la revendication 6, **caractérisé en ce que** le polypeptide a été préparé au moyen d'une synthèse chimique.

8. Polypeptide selon la revendication 6, **caractérisé en ce que** le polypeptide a été préparé de manière recombinante.

9. Polypeptide selon la revendication 8, **caractérisé en ce que** le fragment polypeptidique se présente sous forme d'une protéine de fusion hétérologue.

10. Polypeptide selon la revendication 8, **caractérisé en ce que** le polypeptide se présente sous forme de protéine de fusion autologue.

11. Polypeptide selon l'une quelconque des revendication 6 à 10, **caractérisé en ce que** les fragments polypeptidiques avec les séquences d'acides aminés correspondant aux régions homologues constituées du domaine N-terminal de la gp116 se trouvent sur une chaîne polypeptidique.

12. Polypeptide selon l'une quelconque des revendication 6 à 10, **caractérisé en ce que** les fragments polypeptidiques individuels sont reliés entre eux par couplage chimique.

13. Ensemble de tests pour détecter des anticorps contre le HCMV, **caractérisé en ce qu'**il comprend un polypeptide selon l'une des revendications 1 à 12.

14. Ensemble de tests selon la revendication 13, **caractérisé en ce qu'**il est approprié à la détection d'anticorps protecteurs contre le HCMV.

15. Ensemble de test selon la revendication 13 ou 14, **caractérisé en ce que** c'est un ensemble de tests pour réaliser un test ELISA.

16. Utilisation d'un polypeptide selon l'une des revendications 1 à 12 dans le domaine diagnostique.

17. Utilisation selon la revendication 16, **caractérisée en ce qu'**un polypeptide selon l'une des revendications 1 à 12 est utilisé pour cribler du plasma humain ou pour sélectionner des dons de plasma pour la préparation d'hyperimmunoglobuline de HCMV.

18. Vaccin pour la prophylaxie d'une infection par le HCMV, **caractérisé en ce que** le vaccin comprend un polypeptide selon l'une des revendications 1 à 12.

19. Utilisation d'un polypeptide selon l'une des revendications 1 à 12 pour la préparation d'un vaccin, qui agit à titre prophylactique contre des infections par le HCMV.
